(19) Europäisches Patentamt
European Patent Office
Office européen des brevets

(11) Numéro de publication: **0 017 578**
**B1**

(12) **FASCICULE DE BREVET EUROPEEN**

(45) Date de publication du fascicule du brevet: **23.03.83**

(51) Int. Cl.³: **C 07 D 311/24**, A 61 K 31/35

(21) Numéro de dépôt: **80400430.7**

(22) Date de dépôt: **01.04.80**

(54) **Acides 5-(2-hydroxy-3-thiopropoxy)chromone-2-carboxyliques, leurs sels et esters, procédé de préparation et médicaments.**

(30) Priorité: **06.04.79 FR 798859**

(43) Date de publication de la demande: **15.10.80 Bulletin 80/21**

(45) Mention de la délivrance du brevet: **23.03.83 Bulletin 83/12**

(84) Etats contractants désignés: **AT BE CH DE FR GB IT LI LU NL SE**

(56) Documents cités:
**FR-A-2 120 126**
**GB-A-1 093 673**

(73) Titulaire: **SANOFI, société anonyme, 40, Avenue George V, F-75008 Paris (FR)**

(72) Inventeur: **Pedrazzoli, Andrea, Via Anelli 1, Milano (IT)**
Inventeur: **Boveri, Sergio, Via Cesare Abba 5, Monza-Milano (IT)**

(74) Mandataire: **de Haas, Michel et al, Cabinet Beau de Loménie 55 rue d'Amsterdam, F-75008 Paris (FR)**

**EP 0 017 578 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

Acides 5-(2-hydroxy-3-thiopropoxy)chromone-2-carboxyliques, leurs sels et esters, procédé de préparation et médicaments

La présente invention concerne de nouveaux acides 5-(2-hydroxy-3-thiopropoxy)chromone-2-carboxyliques ayant des propriétés anti-anaphylactiques et anti-allergiques, ainsi qu'on procédé pour leur préparation et des médicaments.

Plus particulièrement, la présente invention se réfère à des acides 5-(2-hydroxy-3-thiopropoxy)chromone-2-carboxyliques, caractérisés par la formule générale suivante:

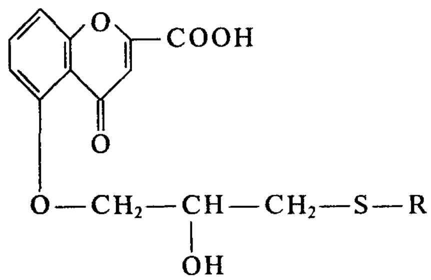

(I)

dans laquelle R représente un groupe alkyle ayant 1 à 16 atomes de carbone, un groupe alkényle ayant 3 à 4 atomes de carbone, un groupe cycloalkyle ayant 3 à 8 atomes de carbone, ou un groupe cycloalkylalkyle ayant 4 à 9 atomes de carbone, ainsi qu'à leurs sels avec des métaux alcalins et alcalino-terreux et à leurs esters alkyliques ayant 1 à 4 atomes de carbone.

Le brevet GB-A-1 093 673 décrit une série d'acides 5-alkoxychromone-2-carboxyliques, différemment substitués dans le groupement alkoxy, caractérisés par la formule générale suivante:

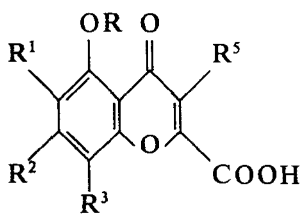

(II)

dans laquelle $R^1$, $R^2$, $R^3$ et $R^5$ représentent de l'hydrogène ou d'autres substituants et R représente un groupe alkyle substitué par un ou plusieurs groupes hydroxy, libres ou estérifiés, ou substitué par un groupe carboxy, un groupe alkyle ou aralkyle interrompu par un ou plusieurs atomes d'oxygène et éventuellement substitué avec on ou plusieurs groupes hydroxy libres ou estérifiés; un groupe O-hétérocycle éventuellement substitué par un ou plusieurs groupes alkyle ou par un ou plusieurs groupes hydroxy, libres ou estérifiés; ou un groupe alkyle substitué par un groupe O-hétérocycle éventuellement substitué par un ou plusieurs groupes alkyle; ainsi que leurs sels, esters et amides. Ledit brevet ne vise pas des chromones portant en position 5 un groupe alkoxy substitué par un groupement thio.

Le brevet FR-A-1 604 165 décrit une série d'acides alkoxychromone-2-carboxyliques, différemment substitués dans le groupement alkoxy, caractérisés par la formule générale suivante:

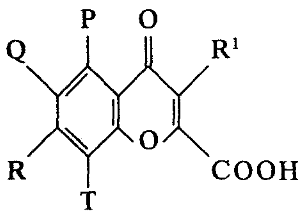

(III)

dans laquelle P est de l'hydrogène ou un groupement autre que OY et Q, R, T représentent un atome d'hydrogène ou un substituant autre que l'hydrogène, un des substituants Q, R, T étant un groupe OY, où Y est un groupe alkyle substitué par un ou plusieurs groupes hydroxy ou carboxy; un groupe alkyle ou aralkyle dans lequel un ou plusieurs groupes méthylène sont remplacés par un atome d'oxygène ou de soufre ou par un groupe carbonyle et peuvent être substitués par un ou plusieurs groupes hydroxy ou carboxy; un groupe hétérocycle renfermant des atomes de carbone et d'oxygène éventuellement substitué par un ou plusieurs groupes hydroxy ou alkyle; ou un groupe alkyle substitué par un ou plusieurs groupes hétérocycliques. Bien que la formule III ci-dessus renferme, d'une façon générale, des chromones substituées dans le noyau benzène par un groupe alkoxy substitué par un groupement thio, ledit groupement n'est jamais dans la position 5. En outre, aucune chromone substituée dans le noyau benzène par un groupe thioalkoxy n'est décrite dans le texte du brevet ci-dessus.

On a maintenant trouvé que les acides 5-(2-hydroxy-3-thiopropoxy)chromone-2-carboxyliques de formule I ci-dessus, leurs sels et esters en $C_1-C_4$ sont doués d'une activité anti-anaphylactique même supérieure à celle des produits correspondants décrits dans le brevet GB-A-1 093 673 ayant dans la molécule un atome d'oxygène au lieu d'un atome de soufre et à celle des produits correspondants compris dans la formule générale du brevet FR-A-1 604 165 ayant le groupement thio-alkoxy dans une position autre que la position 5 du noyau chromone.

Les acides 5-(2-hydroxy-3-thiopropoxy)chromone-2-carboxyliques, leurs sels alcalins et leurs esters sont préparés en traitant un oxalate alkylique inférieur de formule $(COOR^\circ)_2$, où R° est un groupe alkyle ayant 1 à 4 atomes de carbone, avec une 6-(2-hydroxy-3-thiopropoxy)-2-hydroxyacétophénone de formule:

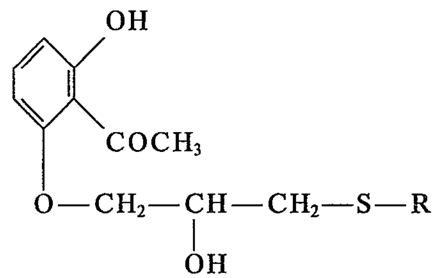

(IV)

dans laquelle R a la signification donnée ci-dessus.

La réaction est conduite en présence d'un agent de condensation alcalin, tel qu'un métal alcalin ou l'hydrure, l'amidure ou l'alcoolate d'un métal alcalin, par exemple le méthylate ou l'éthylate de sodium, l'amidure de sodium ou le sodium métallique, dans solvant organique inerte, tel que le dioxanne, le Méthanol, l'éthanol, le benzène ou leurs mélanges, à la température de reflux du solvant employé.

Suivant un mode d'exécution préféré, la condensation est effectuée dans le benzène en présence de l'alcool R° OH et de l'alcoolate R° ONa correspondant à l'oxalate employé.

Le mélange réactionnel est ensuite acidifié par de l'acide chlorhydrique de préférence et chauffé au reflux pendant 10—30 min. On provoque ainsi une cyclisation avec élimination d'une molécule d'alcool R° OH et formation d'un composé de formule:

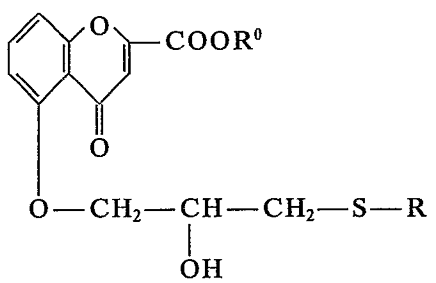

(V)

dans laquelle R et R° ont la signification donnée ci-dessus. Le produit ainsi obtenu est isolé du mélange réactionnel suivant des techniques habituelles.

Les 5-(2-hydroxy-3-thiopropoxy)chromone-2-carboxylates d'alkyle inférieur de formule V ainsi obtenus peuvent ensuite être transformés dans les acides libres correspondants (formule I) par hydrolyse en milieu alcalin, par exemple par chauffage au reflux pendant 10—30 min dans de l'éthanol ou du méthanol, en présence d'hydroxyde de sodium ou de potassium. A la fin de la réaction, on peut isoler le sel de sodium ou de potassium correspondant ou bien l'acide libre par acidification successive.

La préparation des acides libres est effectuée de préférence en faisant réagir sur les 6-(2-hydroxy-3-thiopropoxy)-2-hydroxyacétophénones de formule IV un oxalate de méthyle ou d'éthyle. De cette manière, la cyclisation est favorisée par l'élimination d'une molécule d'alcool de bas point d'ébullition et l'on obtient ainsi des meilleurs rendements en ester V ($R^\circ = CH_3, C_2H_5$) dans l'étape précédant la réaction d'hydrolyse.

Les produits de formule I, sous forme d'acides libres, peuvent être transformés dans leurs sels non toxiques et pharmaceutiquement aceptables suivant les méthodes conventionnelles, par exemple en faisant réagir des quantités stoechiométriques de l'hydroxyde du métal alcalin ou alcalino-terreux et de l'acide libre, en solution aqueuse. Ils sont isolés par évaporation et cristallisation.

Les 6-(2-hydroxy-3-thiopropoxy)-2-hydroxyacétophénones de formule IV utilisées comme composés de départ dans la préparation des acides 5-(2-hydroxy-3-thiopropoxy)chromone-2-carboxyliques de la présente invention sont des produits nouveaux qui peuvent être préparés en faisant réagir la (3-chloro-2-hydroxypropoxy)-2-hydroxyacétophénone avec un excès d'un sel alcalin du mercaptan R—SH dans du benzène ou du toluène, à une température de 90 à 120°C et en les isolant selon des

méthodes connues.

Les acides 5-(2-hydroxy-3-thiopropoxy)chromone-2-carboxyliques de la présente invention ainsi que leurs sels et leurs esters, inhibent la libération et/ou l'action de produits toxiques ou spasmogènes provenant de réactions antigène-anticorps, comme il a été démontré par des tests d'activité anti-anaphylactique.

Les acides 5-(2-hydroxy-3-thiopropoxy)chromone-2-carboxyliques, leurs sels et leurs esters ont été testés chez le rat par voie orale pour évaluer leur capacité d'inhiber l'anaphylaxie cutanée passive dans des animaux sensibilisés à l'ovalbumine et à Bordetella pertussis.

Comme produits de référence, on a employé:

- produit A: acide 7-(2-hydroxyéthoxy)-9-oxoxanthène-2-carboxylique, décrit en Brit. Med. J. 2, 93—95, 1974;
- produit B: acide 5-acétylsalicylique décrit dans le brevet DE-A-2 542 225;
- produit C: 5-(2-hydroxy-3-méthoxypropoxy)chromone-2-carboxylate d'éthyle, décrit dans le brevet GB-A-1 093 673;
- produit D: acide 7-(2-hydroxy-3-méthylthiopropoxy)chromone-2-carboxylique, produit nouveau, compris dans la formule générale du brevet FR-A-1 604 165, mais pas spécifiquement décrit, qui a été préparé de la façon suivante:

On chauffe pendant 8 h à 100° C externes un mélange de 4,70 g de 7-hydroxychromone-2-carboxylate d'éthyle et 20 ml de 1-chloro-2,3-époxypropane en présence de 0,2 ml de pipéridine. On évapore sous pression réduite et on reprend le résidu par 15 ml d'acétate d'éthyle. Après filtration, on évapore la solution sous pression réduite, on reprend le résidu dans 30 ml de chloroforme et 10 ml d'acide chlorhydrique N, on concentre et on agite pendant 1 h. On lave à l'eau la couche organique, on la sèche sur du sulfate de sodium anhydre et on l'évapore. Le résidu, cristallisé dans de l'éthanol, donne 4 g de 7-(3-chloro-2-hydroxypropoxy)chromone-2-carboxylate d'éthyle. On dissout 1,65 g du produit ainsi obtenu dans 40 mg de chlorure de méthylène et, à la solution ainsi obtenue, on ajoute 0,2 g de bromure d'hexadécyltributylphosphonium. On ajoute, à la solution organique, une solution de $CH_3SNa$ dans 20 ml d'eau. On agite 2 h à la température ambiante, on sépare la couche aqueuse et on l'acidifie avec de l'acide chlorhydrique N. Le précipité qui se forme, qui est constitué par l'acide 7-(2-hydroxy-3-méthylthiopropoxy)chromone-2-carboxylique, est filtré et séché; F. 200—202° C.

Les pourcentages d'inhibition, par rapport aux contrôles, de la réaction allergique provoquée par anaphylaxie cutanée passive chez des rats traités par 0,1 ml d'un antisérum homologue dilué 1/10 et injecté par voie intradermique sont résumés dans le tableau I ci-dessous.

Tableau I

| Produit | Dose (mg/kg) os | Δ % par rapport aux contrôles | $DL_{50}$ souris (mg/kg) os |
|---|---|---|---|
| exemple 1 | 10 | −65,55°°) | >1000 |
| | 100 | −75,12°°) | |
| exemple 2 | 50 | −73,00°°) | >1000 |
| | 100 | −75,77°°) | |
| exemple 3 | 100 | −69,12°°) | >1000 |
| exemple 4 | 100 | −75,00°°) | >1000 |
| exemple 5 | 50 | −47,56°°) | >1000 |
| | 100 | −70,84°°) | |
| *exemple 6. b | 100 | −55,37°°) | >1000 |
| Produit A | 100 | −66,19°°) | ≃1000 |
| Produit B | 100 | −29,17°) | ≃1000 |

°) Significatif P ≤ 0,05.
°°) Significatif P ≤ 0,01.

Il ressort de ce tableau que les produits représentatifs de la présente invention ont un pouvoir d'inhibition remarquable même à des doses très faibles, généralement supérieur à celui des produits A et B de référence.

Le tableau II ci-dessous montre les pourcentages d'inhibition après administration orale d'un composé représentatif de la présente invention le produit de l'exemple 1, en comparaison avec le produit C de référence, qui est le dérivé correspondant ayant, dans la chaîne 2-hydroxypropoxy en positon 5 de la chromone, un groupe méthoxy au lieu d'un groupe méthylthio.

Tableau II

| Produit | Dose (mg/kg) os | Δ % par rapport aux contrôles |
|---|---|---|
| exemple s | 10 | −65,55°°) |
| Produit C | 10 | +8,71*) |

*) Non significatif.
°°) Significatif P ≤ 0,01.

De ce tableau, il ressort que le produit représentatif de la présente invention provoque une inhibition de l'anaphylaxie cutanée passive à une dose de 10 mg/kg, alors que le produit de référence est inactif à la même dose.

Le tableau III ci-après montre les pourcentages d'inhibition après adminstration orale d'un composé représentatif de la présente invention le produit de l'exemple 6 a en comparaison avec le produit D de référence, qui est son isomère dans la position 7 du novau chromone.

Tableau III

| Produit | Dose (mg/kg) os | Δ % par rapport aux contrôles |
|---|---|---|
| exemple 6 a. | 10 | −52,95°°) |
| | 100 | −70,40°°) |
| Produit D | 10 | +17,83*) |
| | 100 | −16,17*) |
| | 200 | −30,07°) |

*) Non significatif.
°) Significatif P ≤ 0,05.
°°) Significatif P ≤ 0,01.

Il ressort de ce tableau que le produit représentatif de la présente invention provoque une inhibition importante de l'anaphylaxie cutanée passive lorsqu'il est administré à une dose orale de 10 mg/kg, tandis que le produit de référence est presque inactif, même à des doses dix fois supérieures.

La capacité des acides 5-(2-hydroxy-3-thiopropoxy)chromone-2-carboxyliques de la présente invention, de leurs sels et de leurs esters d'inhiber — aussi bien par voie orale que par voie intraveineuse — l'anaphylaxie cutanée passive chez des rats sensibilisés à l'ovalbumine et à Bordetella pertussis a été comparée à celle de deux produits de référence:

— produit E: sel de sodium du 1,3-bis-(2-carboxy-5-chromonyloxy)-propane-2-ol, bien connu dans sa dénomination commune internationale »chromoglicate de sodium«;

– produit F: acide 5-(2-hydroxy-3-p-cyanophénoxypropoxy)chromone-2-carboxylique décrit dans le brevet US-A-3 953 604.

Le tableau IV ci-après montre les $DE_{50}$ de deux composés représentatifs de la présente invention le produit de l'exemple 1 et produit de l'exemple 6a, en comparaison avec les produits E et F de référence.

Tableau IV

| Produit | $DE_{50}$ (mg/kg) | |
|---|---|---|
| | per os | i.v. |
| exemple 1 | 5,39 | 0,11 |
| exemple 6 a. | 38,11 | 0,13 |
| Produit E | inactif | 1,15 |
| Produit F | inactif | 0,29 |

De ce tableau, il ressort que les produits de la présente invention, contrairement aux produits de référence, sont actifs par voie orale et que leur activité par voie intraveineuse est environ trois fois supérieure à celle du produit F et dix fois supérieure à celle du cromoglicate de sodium.

Grâce à leur activité anti-anaphylactique et antiallergique, les 5-(2-hydroxy-3-thiopropoxy)chromones de la présente invention peuvent être utiles pour le traitement, par exemple, de l'asthme d'origine allergique, du rhume des foins et, en général, des états dus principalement à la combinaison d'un antigène spécifique avec un anticorps réaginique.

En tant que médicaments, les produits de la présente invention peuvent être administrés par la voie orale ou parentérale ou par inhalation, soit seuls, soit sous forme de préparations pharmaceutiques appropriées, telles que des comprimés, des poudres, des granules, des capsules, des élixirs, des suspensions, des sirops et des solutions ou des suspensions pour injection ou pour inhalation.

Les préparations pharmaceutiques destinées à l'administration par voie orale contiennent, outre la substance active, un ou plusieurs excipients organiques ou minéraux acceptables du point de vue pharmaceutique et compatibles avec le principe actif, ainsi que des édulcorants, des aromatisants, des colorants, des agents de conservation et similaires.

Pour la préparation des comprimés, on pourra utilisier, comme excipients, des diluants inertes, comme le carbonate de calcium, le carbonate de sodium, le lactose, le talc, des agents de granulation et de désagrégation comme l'amidon et l'acide alginique, des liants comme l'amidon, la gélatine et la gomme arabique, des agents lubrifiants comme le stéarate de magnésium et l'acide stéarique. Les comprimés peuvent être revêtus ou non. Le revêtement a pour but de retarder la décomposition et l'absorption de la substance active dans le tractus gastro-intestinal et de produire ainsi un effet retard prolongé. Les suspensions, les sirops et les élixirs peuvent contenir, outre la substance active, des agents de suspension, tels que la méthylcellulose, la gomme adragante, l'alginate de sodium et similaires, des mouillants, tels que la lécithine, le stéarate de polyoxyéthylène, le mono-oléate de polyoxyéthylène-sorbitanne et des agents de conservation, tels que le p-hydroxybenzoate d'éthyle. Les gélules peuvent contenir la substance active soit seule, soit en mélange avec des diluants inertes solides, comme par exemple le carbonate de calcium, le phosphate de calcium et le kaolin.

Pour l'administration par inhalation, les compositions selon l'invention peuvent se présenter sous la forme d'une poudre ou d'un spray aérosol.

Les composition susdites peuvent également renfermer d'autres produits actifs tels que, par exemple, des bronchodilatateurs, des antitussifs, des antihistaminiques ou des anxiolytiques.

Les acides 5-(2-hydroxy-3-thiopropoxy)chromone-2-carboxyliques, ainsi que leurs sels et leurs esters $C_1-C_4$ peuvent être administrés par voie orale à des doses variables entre 5 et 1000 mg ou par inhalation à une dose de 0,5–15 mg. Ils sont administrés de préférence dans les compositions pharmaceutiques de la présente invention, sous forme d'unité de dosage comprenant de 5 à 250 mg de principe actif, mélangés avec un excipient pharmaceutique comme spécifié ci-dessus.

Les exemples suivants illustrent l'invention sans toutefois la limiter. Dans les exemples, ainsi que dans la partie descriptive et dans les revendications, les produits sont désignés »acides chromone-2-carboxyliques«. Il est entendu que la nomemclature des produits de la présente invention est la suivante: »acides 5-(2-hydroxy-3-hydroxypropoxy)-4-oxo-4H-chromène-2-carboxyliques« et que le terme »chromone«, utilisé pour simplifier, désigne le »4-oxo-4H-chromène«.

## Preparation

On ajoute à une solution froide de 5,2 g d'hydroxyde de sodium et 6,3 g de méthylmercaptan dans 70 ml d'eau une solution de 24,5 g de 2-hydroxy-6-(3-chloro-2-hydroxypropoxy)acétophénone et 0,2 g de bromure d'hexadécyltributylphosphonium dans 240 ml de toluène. On laisse le mélange sous agitation à 25° C pendant 2 h, puis on l'acidifie par de l'acide chlorhydrique N. On sépare le toluène, on extrait la couche aqueuse par 30 ml de toluène, on lave à l'eau la phase organique, on la sèche sur sulfate de sodium et on l'évapore jusqu'à siccité. On reprend le résidu dans 30 ml d'éther diisopropylique et on le refroidit. Après filtration et purification, on obtient 24 g 2-hydroxy-6-(2-hydroxy-3-méthylthiopropoxy)acétophénone; F. 63 à 65° C.

Da la même façon, en faisant réagir la 2-hydroxy-6-(3-chloro-2-hydroxypropoxy)acétophénone avec de l'éthylmercaptan, du propylmercaptan, de l'isopropylmercaptan, du butylmercaptan et, respectivement, de l'hexylmercaptan, on obtient la 2-hydroxy-6-(2-hydroxy-3-éthylthiopropoxy)acétophénon; la 2-hydroxy-6-(2-hydroxy-3-propylthiopropoxy)acétophénone; la 2-hydroxy-6-(2-hydroxy-3-isopropylthiopropoxy)acétophénone; la 2-hydroxy-6-(2-hydroxy-3-butylthiopropoxy)acétophénone; et, respectivement, la 2-Hydroxy-6-(2-hydroxy-3-hexylthiopropoxy)acétophénone.

## Exemple 1

On ajoute à une solution de 3,3 g de sodium dans 80 ml d'éthanol et 80 ml de benzène anhydres un mélange de 15,7 g de 2-hydroxy-6-(2-hydroxy-3-méthylthiopropoxy)acétophénone, 28,5 g d'oxalate diéthylique et 40 ml de benzène. On chauffe le mélange réactionnel au reflux 5 h sous agitation, on le refroidit à 20—30° C et on l'acidifie avec de l'acide chlorhydrique en éthanol. Après 15 min de chauffage au reflux, on refroidit, on évapore la solution limpide jusqu'à siccité, on lave le résidu avec de l'hexane, puis on le dissout dans 150 ml d'acétate d'éthyle. On lave à l'eau la solution ainsi obtenue et on réduit son volume à 20—25 ml par évaporation. Après purification sur une colonne d'alumine, on évapore le solvant et on obient une huile qui cristallise lentement. On reprend le produit dans de l'éther diisopropylique et on filtre. Après cristallisation du produit brut ainsi obtenu dans un mélange de 15 ml d'acétate d'éthyle et 40 ml d'éther diisopropylique, on obtient 9 g de 5-(2-hydroxy-3-méthylthiopropoxy)chromone-2-carboxylate d'éthyle; F. 69—71° C.

## Exemples 2 à 5

En opérant comme illustré à l'exemple 1, par réaction des acétophénones décrites dans la préparation avec de l'oxalate diéthylique et par acidification avec de l'acide chlorhydrique, on obtient les esters donnés dans le tableau V ci-dessous.

Tableau V

| Exemples | Produits |
|---|---|
| 2 | 5-(2-hydroxy-3-éthylthiopropoxy)-chromone-2-carboxylate d'éthyle; F. 66 à 68°C (cristallisé dans de l'éther diisopropylique) |
| 3 | 5-(2-hydroxy-3-propylthiopropoxy)chromone-2-carboxylate d'éthyle; F. 63 à 66°C (cristallisé dans le l'éther diisopropylique) |
| 4 | 5-(2-hydroxy-3-isopropylthiopropoxy)chromone-2-carboxylate d'éthyle; F. 66 à 69°C (cristallisé dans de l'éther diisopropylique) |
| 5 | 5-(2-hydroxy-3-butylthiopropoxy)-chromone-2-carboxylate d'éthyle; F. 50 à 52°C (cristallisé dans de l'éther diisopropylique) |

Exemple 6

a) On ajoute à une solution de 3,4 g de 5-(2-hydroxy-3-méthylthiopropoxy)chromone-2-carboxylate d'éthyle dans 20 ml d'acétone 11 ml d'hydroxyde de sodium N et on chauffe le mélange à 50° C pendant 10 min. On évapore le solvant sous pression réduite, on ajoute 30 ml d'eau, on extrait par de l'éther diéthylique, on filtre sur du charbon et on acidifie la solution limpide ainsi obtenue par de l'acide chlorhydrique. On filtre le produit qui précipite, on le lave à l'eau et on le sèche. On obtient 2,8 g d'acide 5-(2-hydroxy-3-méthylthiopropoxy)chromone-2-carboxylique dihydraté; F. 76 à 79° C.

b) De la même façon, en hydrolysant le 5-(2-hydroxy-3-butylthiopropoxy)chromone-2-carboxylate d'éthyle, on obtient l'acide 5-(2-hydroxy-3-butylthiopropoxy)chromone-2-carboxylique monohydraté; F. 58 à 61° C.

Exemple 7

On agite un mélange de 3,7 g d'acide 5-(2-hydroxy-3-butylthiopropoxy)chromone-2-carboxylique monohydraté et 10 ml d'hydroxyde de sodium N pendant 10 min, puis on filtre le précipité qui se forme, on le lave avec peu d'acétone et on le sèche. On obtient ainsi 3,5 g de 5-(2-hydroxy-3-butylthiopropoxy)chromone-2-carboxylate de sodium.

Exemple 8

On ajoute à une solution de 2,7 g de sodium dans 60 ml d'éthanol et 60 ml de benzène anhydres un mélange de 15,2 g de 2-hydroxy-6-(2-hydroxy-3-hexylthiopropoxy)acétophénone, 21 g d'oxalate diéthylique et 30 ml de benzène. On chauffe le mélange réactionnel au reflux pendant 5 h sous agitation, puis on le refroidit à 20—30° C et on l'acidifie avec de l'acide chlorhydrique en éthanol. Après 15 min de chauffage au reflux, on refroidit, on évapore la solution limpide jusqu'à siccité, on lave le résidu avec de l'hexane, puis on le dissout dans 150 ml d'acétate d'éthyle. On lave à l'eau la solution ainsi obtenue et on réduit son volume à 20—25 ml par évaporation. Après purification sur une colonne d'alumine, on évapore le solvant et on obtient une huile qui est dissoute dans 80 ml d'acétone et 36 ml d'hydroxyde de sodium N. On chauffe la solution ainsi obtenue pendant 10 min, puis on la refroidit, on filtre le précipité qui se forme, on le lave avec un mélange eau-acétone et on le dissout dans 500 ml d'eau chaude. On acidifie par de l'acide chlorhydrique, on filtre le précipité, on le lave à l'eau et on le sèche. On obtient 10 g d'acide 5-(2-hydroxy-3-hexylthiopropoxy)chromone-2-carboxylique; F. 97 à 100° C.

Exemples 9 et 10

En opérant comme illustré à l'exemple 1 par réaction de la 2-hydroxy-6-(2-hydroxy-3-méthylthiopropoxy)acétophénone avec de l'oxalate diisopropylique ou n-butylique et par acidification avec de l'acide chlorhydrique, on obtient les esters donnés dans le tableau VI ci-dessous.

Tableau VI

| Exemples | Produits |
|---|---|
| 9 | 5-(2-hydroxy-3-méthylthiopropoxy)chromone-2-carboxylate d'isopropyle; F. 71 à 73°C (cristallisé dans de l'éther diisopropylique) |
| 10 | 5-(2-hydroxy-3-méthylthiopropoxy)chromone-2-carboxylate de n-butyle huile jaune; $n_D^{20} = 1,5648$. L'huile jaune obtenue cristallisé spontanément au bout de quelques jours et fond alors à 52—54°C |

**Revendications**

1. Acides 5-(2-hydroxy-3-thiopropoxy)chromone-2-carboxyliques, caractérisés par la formule générale:

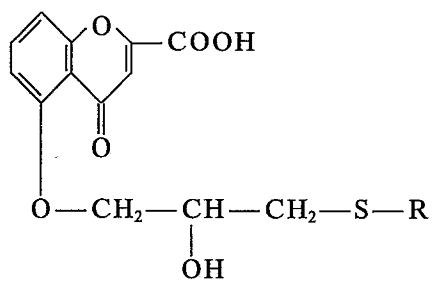

dans laquelle R représente un groupe alkyle ayant 1 à 16 atomes de carbone, un groupe alkényle ayant 3 à 4 atomes de carbone, un groupe cycloalkyle ayant 3 à 8 atomes de carbone ou un groupe cycloalkylalkyle ayant 4 à 9 atomes de carbone; leurs sels avec des métaux alcalins ou alcalino-terreux et leurs esters alkyliques ayant 1 à 4 atomes de carbone.

2. Acides 5-(2-hydroxy-3-méthylthiopropoxy)chromone-2-carboxyliques, leurs sels alcalins ou alcalino-terreux et leurs esters alkyliques ayant de 1 à 4 atomes de carbone.

3. 5-(2-hydroxy-3-méthylthiopropoxy)chromone-2-carboxylate d'éthyle.

4. Procédé pour la préparation d'acides 5-(2-hydroxy-3-thiopropoxy)chromone-2-carboxyliques de formule:

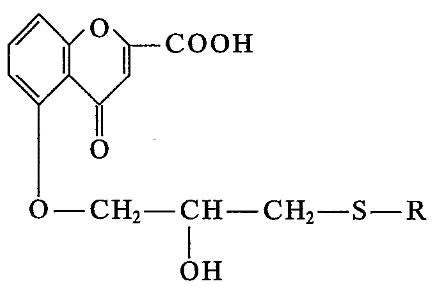

dans laquelle R représente un groupe alkyle ayant 1 à 16 atomes de carbone, un groupe alkényle ayant 3 à 4 atomes de carbone, un groupe cycloalkyle ayant 3 à 8 atomes de carbone ou un groupe cycloalkylalkyle ayant 4 à 9 atomes de carbone, leurs sels avec des métaux alcalins ou alcalino-terreux et leurs esters alkyliques ayant 1 à 4 atomes de carbone, caractérisé en ce que l'on traite un oxalate dialkylique inférieur de formule $(COOR°)_2$, dans laquelle R° est un groupe alkyle ayant 1 à 4 atomes de carbone, avec une 2-hydroxy-6-(2-hydroxy-3-thiopropoxy)-acétophénone de formule:

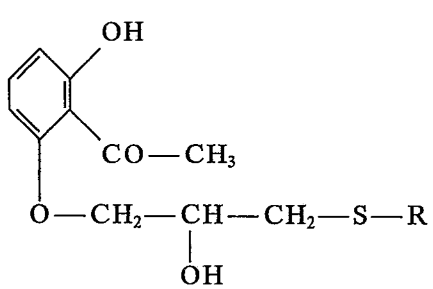

dans laquelle R a la signification donnée ci-dessus, en présence d'un agent de condensation alcalin, la réaction étant conduite dans un solvant organique inerte et à la température de reflux du solvant utilisé, on traite ensuite le mélange réactionnel par un acide puis le mélange est chauffé à la température de reflux du solvant et, éventuellement on hydrolyse l'ester de l'acide 5-(2-hydroxy-3-thiopropoxy)chromone-2-carboxylique ainsi obtenu de formule:

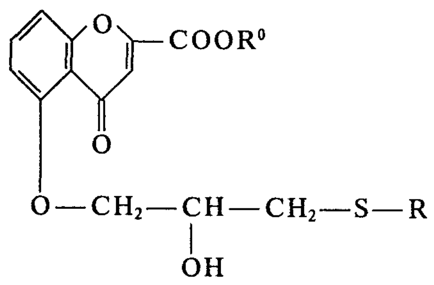

dans laquelle R et R° ont les significations données ci-dessus et on tranforme éventuellement l'acide ainsi obtenu dans ses sels alcalins ou alcalino-terreux.

5. Procédé selon la revendication 4, caractérisé en ce qu'on conduit la réaction de la 6-(2-hydroxy-3-thiopropoxy)-acétophénone avec l'oxalate dialkylique de formule $(COOR°)_2$, où R° a la signification donnée dans la revendication 4, dans le en présence de l'alcool R°OH et de l'alcoolate R°ONa correspondant à l'oxalate employé.

6. Procédé selon les revendications 4 et 5, caractérisé en ce que l'oxalate dialkylique de formule $(COOR°)_2$ est l'oxalate diéthylique.

7. Composition pharmaceutique sous forme d'unité de dosage, caractérisée en ce qu'elle renferme, en tant que principe actif, de 5 à 250 mg d'un composé tel que revendiqué dans les revendications 1 à 3, mélangé avec un excipient pharmaceutique.

**Patentansprüche**

1. 5-(2-Hydroxy-3-thiopropoxy)-chromon-2-carbonsäuren, gekennzeichnet durch die allgemeine Formel

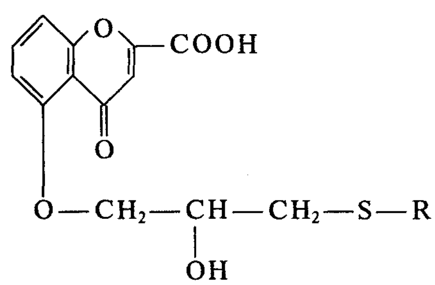

worin R eine Alkylgruppe mit 1 bis 16 Kohlenstoffatomen, eine Alkenylgruppe mit 3 bis 4 Kohlenstoffatomen, eine Cycloalkylgruppe mit 3 bis 8 Kohlenstoffatomen oder eine Cycloalkylalkylgruppe mit 4 bis 9 Kohlenstoffatomen darstellt, ihre Alkalimetallsalze oder Erdalkalimetallsalze und ihre Alkylester mit 1 bis 4 Kohlenstoffatomen.

2. 5-(2-Hydroxy-3-methylthiopropoxy)-chromon-2-carbonsäuren, ihre Alkali- oder Erdalkalisalze und ihre Alkylester mit 1 bis 4 Kohlenstoffatomen.

3. 5-(2-Hydroxy-3-methylthiopropoxy)-chromon-2-äthylcarboxylat.

4. Verfahren zur Herstellung von 5-(2-Hydroxy-3-thiopropoxy)-chromon-2-carbonsäuren der Formel

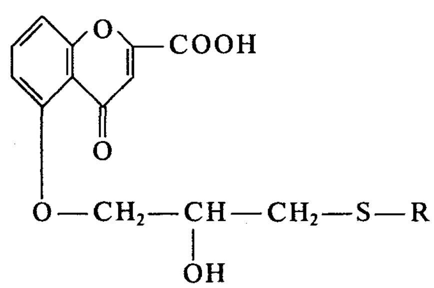

worin R eine Alkylgruppe mit 1 bis 16 Kohlenstoffatomen, eine Alkenylgruppe mit 3 bis 4 Kohlenstoffatomen, eine Cycloalkylgruppe mit 3 bis 8 Kohlenstoffatomen oder eine Cycloalkylalkylgruppe mit 4 bis 9 Kohlenstoffatomen darstellt, über Alkalimetallsalze oder Erdalkalimetallsalze und ihrer Alkylester mit 1 bis 4 Kohlenstoffatomen, dadurch gekennzeichnet, daß ein Niedrigdialkyloxalat der Formel $(COOR°)_2$, worin R° eine Alkylgruppe mit 1 bis 4 Kohlenstoffatomen ist, mit 2-Hydroxy-6-(2-hydroxy-3-thiopropoxy)-acetophenon der Formel

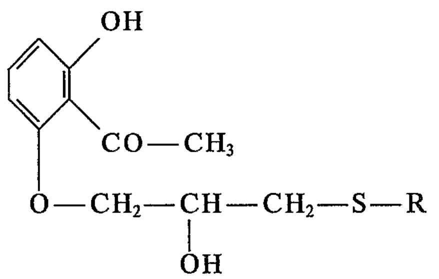

worin R obige Bedeutung hat, in Anwesenheit eines alkalischen Kondensationsmittels behandelt wird, wobei die Umsetzung in einem inerten organischen Lösungsmittel und bei der Rückflußtemperatur des verwendeten Lösungsmittels durchgeführt wird, die Reaktionsmischung danach mit einer Säure behandelt wird, die Mischung dann auf die Rückflußtemperatur des Lösungsmittels erhitzt wird und gegebenenfalls der so erhaltene 5-(2-Hydroxy-3-thiopropoxy)-chromon-2-carbonsäureester der Formel

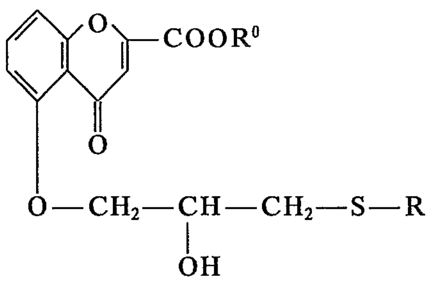

worin R und R° obige Bedeutung haben, hydrolysiert und gegebenenfalls die so erhaltene Säure in ihre Alkali- oder Erdalkalisalze übergeführt wird.

5. Verfahren nach Anspruch 4, dadurch gekennzeichnet, daß die Umsetzung von 6-(2-Hydroxy-3-thiopropoxy)-acetophenon mit Dialkyloxalat der Formel $(COOR°)_2$, worin R° die in Anspruch 4 angegebene Bedeutung hat, in Benzol in Anwesenheit des Alkohols R°OH und des Alkoholates R°ONa entsprechend dem verwendeten Oxalat durchgeführt wird.

6. Verfahren nach den Ansprüchen 4 und 5, dadurch gekennzeichnet, daß das Dialkyloxalat der Formel $(COOR°)_2$ Diäthyloxalat ist.

7. Pharmazeutisches Präparat in Dosiseinheitsform, dadurch gekennzeichnet, daß es als Hauptwirkstoff 5 bis 250 mg einer Verbindung gemäß den Ansprüchen 1 bis 3 in Mischung mit einem pharmazeutischen Träger enthält.

## Claims

1. 5-(2-hydroxy-3-thiopropoxy)chromone-2-carboxylic acids characterized by the general formula

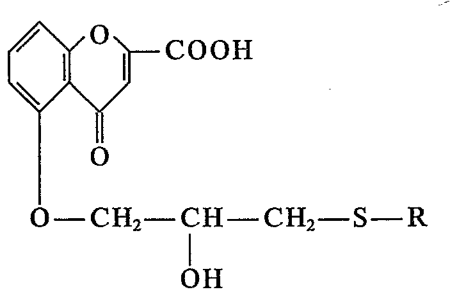

wherein R ist an alkyl group of from 1 to 16 carbon atoms, an alkenyl group of 3 to 4 carbon atoms, a cycloalkyl group of from 3 to 8 carbon atoms or a cycloalkylalkyl group of from 4 to 9 carbon atoms; and alkali and alkaline-earth metal salts thereof and alkyl esters thereof having 1 to 4 carbon atoms.

2. 5-(2-hydroxy-3-methylthiopropoxy)chromone-2-carboxylic acids and the alkali and alkaline-earth metal salts thereof and the alkyl esters thereof having from 1 to 4 carbon atoms.

3. Ethyl 5-(2-hydroxy-3-methylthiopropoxy)chromone-2-carboxylate.

4. A process for the preparation of 5-(2-hydroxy-3-thiopropoxy)chromone-2-carboxylic acids of formula

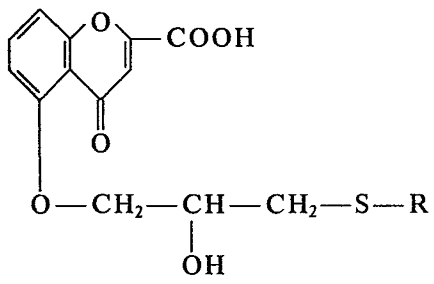

wherein R represents an alkyl group of from 1 to 16 carbon atoms, an alkenyl group of 3 to 4 carbon atoms, a cycloalkyl group of from 3 to 8 carbon atoms or a cycloalkylalkyl group of from 4 to 9 carbon atoms and the alkali and alkaline-earth metal salts thereof and the alkyl esters thereof having 1 to 4 carbon atoms, characterized in that a lower dialkyl oxalate of formula $(COOR°)_2$, where R° is an alkyl group of from 1 to 4 carbon atoms, ist treated with a 6-(2-hydroxy-3-thiopropoxy)acetophenone of formula

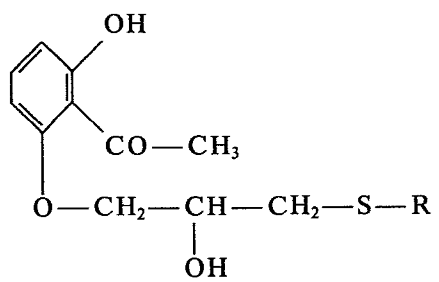

wherein R has the above stated meaning, in the presence of an alkaline condensing agent, the reaction being carried out in an inert organic solvent and at the reflux temperature of the solvent used, and the reaction mixture is treated with an acid, then the mixture is heated to the reflux temperature of the solvent and the 5-(2-hydroxy-3-thiopropoxy)chromone-2-carboxylic acid ester thus obtained of formula

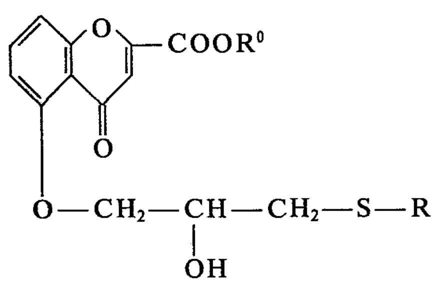

wherein R and R° have the above-stated meaning, is optionally hydrolysed, and the acid thus obtained is optionally converted into its alkali or alkaline-earth metal salts.

5. A process according to claim 4 in which the reaction of the 6-(2-hydroxy-3-thiopropoxy)acetophenone with the dialkyl oxalate of formula $(COOR°)_2$, in which R° has the meaning given in claim 4, is carried out in benzene in the presence of the alcohol R°OH and of the alcoholate R°ONa corresponding to the employed oxalate.

6. A process according to claims 4 and 5, characterized in that the dialkyl oxalate of formula $(COOR°)_2$ is the diethyl oxalate.

7. A pharmaceutical composition in dosage unit form, characterized in that it comprises as active ingredient from 5 to 250 mg of a compound as claimed in claims 1 to 3 in admixture with a pharmaceutical carrier.